(19) Europäisches Patentamt
European Patent Office
Office européen des brevets

(11) Veröffentlichungsnummer: **0 258 618 B1**

(12) # EUROPÄISCHE PATENTSCHRIFT

(45) Veröffentlichungstag der Patentschrift: **06.05.92**

(51) Int. Cl.[5]: **C07F 9/6503**, C07F 9/6539, C07F 9/6518, C07F 9/6527, C07F 9/6536, A61K 31/675

(21) Anmeldenummer: **87110830.4**

(22) Anmeldetag: **25.07.87**

Die Akte enthält technische Angaben, die nach dem Eingang der Anmeldung eingereicht wurden und die nicht in dieser Patentschrift enthalten sind.

(54) **Neue Diphosphonsäurederivate, Verfahren zu deren Herstellung und diese Verbindungen enthaltende Arzneimittel.**

(30) Priorität: **01.08.86 DE 3626058**

(43) Veröffentlichungstag der Anmeldung: **09.03.88 Patentblatt 88/10**

(45) Bekanntmachung des Hinweises auf die Patenterteilung: **06.05.92 Patentblatt 92/19**

(84) Benannte Vertragsstaaten: **AT BE CH DE ES FR GB GR IT LI LU NL SE**

(56) Entgegenhaltungen:
**EP-A- 170 228**
**EP-A- 0 084 822**
**DE-A- 3 203 307**

(73) Patentinhaber: **BOEHRINGER MANNHEIM GMBH**
**Sandhofer Strasse 116**
**W-6800 Mannheim 31(DE)**

(72) Erfinder: **Bosies, Elmar, Dr. rer. nat.**
**Delplstrasse 11**
**W-6940 Weinheim(DE)**
Erfinder: **Gall, Rudi, Dr. phil.**
**Ulmenstrasse 1**
**W-6945 Hirschberg(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.08/2.18/2.0)

## Beschreibung

Die vorliegende Erfindung betrifft neue Diphosphonsäurederivate, Verfahren zu deren Herstellung sowie Arzneimittel, die diese Substanzen enthalten.

In der DE-OS 32 03 307 bzw.DE-OS 32 02 208 sind Aryl-ethan-diphosphonate, z.B. das Thienyl-ethan-diphosphonat bzw. ein Pyrazol-ethan-diphosphonat mit ausgeprägter antiinflammatorischer Wirkung beschrieben.

In der DE-PS 18 13 659 sind Diphosphonsäurederivate beschrieben, von denen die 1-Hydroxy-ethan-1,1-diphosphonsäure als Mittel zur Behandlung von Morbus Paget Bedeutung erlangt hat. In der EP-OS 186 405 sind u. a. Pyridylalkyldiphosphonate und in der DE-OS 34 28 524 sind heteroaromatische Alkyldiphosphonate, bei denen die Alkylenkette mindestens 2 Kohlenstoffatome umfaßt, beschrieben. Es wurde nun gefunden, daß analoge Derivate dieser Verbindungen, in denen zwischen dem Diphosphonatrest und dem heterocyclischen Rest nur 1 Kohlenstoffatom steht und der Heterocyclus nicht Pyrazol bedeutet, auch diese Wirkung zeigen und darüber hinaus als gute Calciumkomplexbildner zur breiteren Behandlung von Calciumstoffwechselstörungen geeignet sind. Sie lassen sich vor allem sehr gut dort einsetzen, wo der Knochen-auf- und -abbau gestört ist, d. h. sie sind geeingent zur Behandlung von Erkrankungen des Skelettsystems wie z. B. Osteoporose, Morbus Bechterew u. a.

Aufgrund dieser Eigenschaften finden sie aber auch Verwendung in der Therapie von Knochenmetastasen, der Urolithiasis und zur Verhinderung heterotoper Ossifikationen. Durch ihre Beeinflussung des Calciumstoffwechsels bilden sie weiterhin eine Grundlage für die Behandlung der rheumatoiden Arthritis, der Osteoarthritis und der degenerativen Arthrose.

Gegenstand der vorliegenden Anmeldung sind demnach Diphosphonate der allgemeinen Formel I

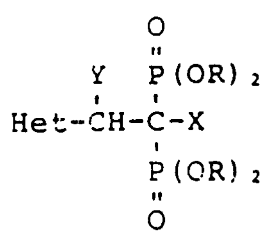

(I)

in der

- Het einen Imidazol-, Imidazolin-, Isoxazol-, Oxazol-, Oxazolin-, Thiazol-, Thiazolin-, Triazol-, Oxadiazol- oder Thiadiazol-, rest, der gegebenenfalls ein- oder mehrfach durch $C_1$-$C_4$-Alkyl, $C_1$-$C_4$-Alkoxy, Phenyl, Cyclohexyl, Cyclohexylmethyl, Halogen oder die Aminogruppe substituiert sein kann,
- Y Wasserstoff oder $C_1$-$C_4$-Alkyl
- X Wasserstoff, Hydroxy oder gegebenfalls durch $C_1$-$C_4$-Alkyl substituiertes Amino und
- R Wasserstoff oder $C_1$-$C_4$-Alkyl

bedeuten.

Sowie deren pharmakologisch unbedenkliche Salze

Alkyl bedeutet bevorzugt den Methyl-, Ethyl- und Isobutylrest.

Asymmetrische Kohlenstoffatome können die R-, S- oder R,S- Konfiguration besitzen.

Verbindungen der allgemeinen Formel I werden nach an sich bekannten Verfahren hergestellt.

Für den Fall, daß X in der allgemeinen Formel I OH bedeutet, stellt man die Substanzen vorzugsweise dadurch her, daß man

a) eine Carbonsäure der allgemeinen Formel II

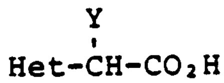

(II),

in der Het und Y die oben angegebenen Bedeutungen haben, mit einem Gemisch aus phosphoriger Säure oder Phosphorsäure und einem Phosphorhalogenid umgesetzt und anschließend zur freien Diphosphonsäure verseift, oder

b) ein Carbonsäurechlorid der allgemeinen Formel III

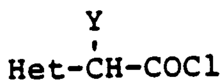

(III),

in der Het und Y die oben angegebenen Bedeutungen haben, mit einem Trialkylphosphit der allgemeinen Formel IV

$P(OR')_3$ (IV),

in der R′ niederes Alkyl bedeutet, zu einem Acylphosphonat der allgemeinen Formel V

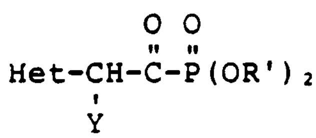

(V),

in der Het, Y und R′ die oben angegebenen Bedeutungen haben,
umsetzt, anschließend mit einem Dialkylphosphit der allgemeinen Formel VI

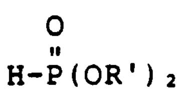

(VI),

in der R′ die oben angegebene Bedeutung hat, zu einem Diphosphonat der allgemeinen Formel VII

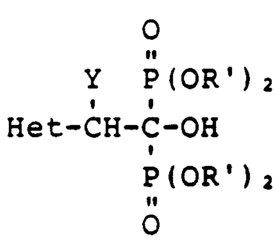

(VII),

in der Het, Y und R′ die oben angegebenen Bedeutungen haben, reagieren läßt und gegebenenfalls die entstandenen Tetraester zu Diestern oder Säuren der allgemeinen Formel I verseift, oder
für den Fall, daß X in der allgemeinen Formel I gegebenenfalls durch Alkylgruppen substituiertes Amino bedeutet,

c) ein Carbonsäurederivat der allgemeinen Formel VIII

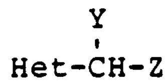

(VIII),

in der Het und Y die oben angegebenen Bedeutungen haben und Z eine Nitril-, Iminoether- oder eine N,N-Dialkylcarboxamidogruppe darstellt,
mit einer Phosphorverbindung der allgemeinen Formel IX

$PT_3$ (IX),

in der T = Halogen, OH oder OR′ bedeutet, wobei R′ die oben angegebene Bedeutung hat, umsetzt und gegebenenfalls anschließend verseift,
oder
für den Fall daß X in der allgemeinen Formel I Wasserstoff bedeutet,
d) eine Verbindung der allgemeinen Formel X

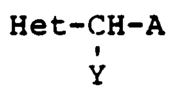

(X),

in der Het und Y die oben angegebenen Bedeutungen haben und A einen reaktiven Rest wie z. B. Halogen oder ein Sulfonat darstellt, mit einer Verbindung der allgemeinen Formel XI

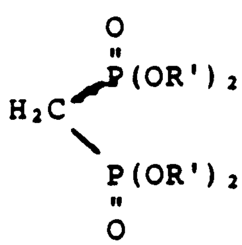

(XI),

in der R′ die oben angegebene Bedeutung hat, zu einem Diphosphonat der allgemeinen Formel XII

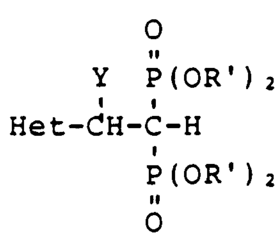

(XII),

in der Het, Y und R′ die oben angegebenen Bedeutungen haben,
umsetzt und gegebenenfalls die entstandenen Tetraester zu Diestern oder Säuren der allgemeinen Formel I verseift.

Die bei Verfahren a) eingesetzten Carbonsäuren der allgemeinen Formel II werden mit 1-2, vorzugsweise 1.5 Mol phosphoriger Säure oder Phosphorsäure und 1-2, vorzugsweise 1.5 Mol Phosphortrihalogenid bei Temperaturen von 80 - 130° C, vorzugsweise 100 - 110° C umgesetzt. Man kann die Reaktion auch in

Gegenwart von Verdünnungsmitteln, wie Halogenkohlenwasserstoffen, insbesondere Chlorbenzol, Tetrachlorethan oder auch Dioxan durchführen. Die anschließende Hydrolyse erfolgt durch Kochen mit Wasser, zweckmäßigerweise jedoch mit halbkonzentrierter Salz- oder Bromwasserstoffsäure.

Bei Verfahren b) läßt man das Säurechlorid der allgemeinen Formel III mit dem Trialkylphospit der allgemeinen Formel IV bei Temperaturen zwischen 0 und 60° C, vorzugsweise bei 20 - 40° C zur Reaktion kommen. Man kann ohne Lösungsmittel oder auch in Gegenwart von inerten Lösungsmitteln wie Diethylether, Tetrahydrofuran, Dioxan oder auch halogenierten Kohlenwasserstoffen, wie z. B. Methylenchlorid arbeiten. Das als Zwischenprodukt entstehende Acylphosphonat der allgemeinen Formel V kann isoliert oder direkt weiter umgesetzt werden.

Die anschließende Reaktion führt man in Gegenwart einer schwachen Base, vorzugsweise einem sec. Amin wie z. B. Dibutylamin bei einer Temperatur von 0 bis 60° C, vorzugsweise bei 10 - 30° C durch.

Bei Verfahren c) setzt man die Nitrile der allgemeinen Formel VIII mit phosphoriger Säure bei Temperaturen von 110 - 180° C um. Die Reaktion kann ohne oder in Gegenwart von aprotischen Lösungsmitteln wie z. B. Diglykoldimethylether oder Diglykoldiethylether durchgeführt werden. Man kann die Nitrile jedoch auch mit einem Phosphortrihalogenid, z. B. Phosphortribromid oder Phosphortrichlorid in einem inerten Lösungsmittel wie z. B. Dioxan oder Tetrahydrofuran gegebenenfalls unter Zustaz von Wasser bei Temperaturen von 20 - 80° C zur Reaktion bringen.Iminoether der allgemeinen Formel VIII läßt man mit Dialkylphosphiten vorzugsweise in Gegenwart äquimolarer Mengen Natrium in inerten Lösungsmitteln wie Diethylether, Dioxan oder auch Benzol reagieren, wobei die Umsetzungen in der Regel bei der Rückflußtemperatur des entsprechenden Lösungsmittels stattfindet. Säureamide der allgemeinen Formel VIII kann man in inerten Lösungsmitteln wie z. B. halogenierten Kohlenwasserstoffen oder Ethern wie z. B. Diethylether mit einem Gemisch aus Phosphorpentahalogenid / phosphoriger Säure oder auch Oxalylchlorid / Trialkylphosphit umsetzen.

Bei Verfahren d) setzt man den Methylendiphosphonsäureester der allgemeinen Formel XI in Form seines Natrium- oder Kaliumsalzes ein. Hierzu wird er mit Natrium, Kalium oder dem entsprechenden Hydrid in einem inerten Lösungsmittel wie z. B. Benzol, Toluol oder Dimethylformamid bei einer Temperatur von 0 bis 40° C vorzugsweise bei 25° C umgesetzt. Das Alkalisalz wird ohne Isolierung mit dem entsprechenden Halogenid bzw. Sulfonat zur Reaktion gebracht. Die Temperatur liegt hierbei bei 20 - 110° C.

Die bei den Verfahren b), c) und d) gegebenenfalls anfallenden Tetraalkylester können zu Diestern oder den freien Tetrasäuren verseift werden. Die Verseifung zu Diestern geschieht in der Regel dadurch, daß man den Tetraalkylester mit einem Alkalihalogenid, vorzugsweise Natriumjodid in einem geeigneten Lösungsmittel wie z. B. Aceton bei Zimmertemperatur behandelt.

Hierbei entsteht das symmetrische Diester / Dinatriumsalz, das gegebenenfalls durch einen sauren Ionenaustauscher in die Diester/Disäure umgewandelt werden kann. Die Verseifung zu freien Diphosphonsäuren geschieht in der Regel durch Kochen mit Salz- oder Bromwasserstoffsäure. Man kann jedoch auch eine Spaltung mit Trimethylsilylhalogenid, vorzugsweise dem Bromid oder Jodid vornehmen. Die freien Diphosphonsäuren können umgekehrt durch Kochen mit Orthoameisensäurealkylestern wieder in die Tetraalkylester überführt werden. Die freien Diphosphonsäuren der allgemeinen Formel I können als freie Säuren oder in Form ihrer Mono- oder Dialkali- bzw. Ammoniumsalze isoliert werden. Die Alkalisalze lassen sich in der Regel durch Umfällen aus Wasser / Methanol oder Wasser / Aceton gut reinigen.

Die Verbindungen der allgemeinen Formel I können gegebenenfalls nachträglich ineinander überführt werden. Sie können z. B. alkyliert werden oder, wenn X in der allgemeinen Formel I eine unsubstituierte Aminogruppe bedeutet, durch Diazotieren in die Verbindungen der allgemeinen Formel I mit X = OH umgewandelt werden. Durch hydrogenolytische Abspaltung einer N-Benzylgruppe lassen sich z. B. die entsprechenden unsubstituierten Verbindungen der allgemeinen Formel I darstellen.

Als pharmakologisch verträgliche Salze werden vor allem Alkali-oder Ammoniumsalze verwendet, die man in üblicher Weise z. B. durch Neutralisation der Verbindungen mit anorganischen oder organischen Basen wie z. B. Natrium- oder Kaliumhydrogencarbonat, Natronlauge, Kalilauge, wässrigem Ammoniak oder Aminen wie z. B. Trimethyl- oder Triethylamin herstellt.

Die erfindungsgemäßen neuen Substanzen der Formel I und ihre Salze können in flüssiger oder fester Form enteral und parenteral appliziert werden. Hierbei kommen alle üblichen Applikationsformer in Frage, beispielsweise Tabletten, Kapseln, Dragees, Sirupe, Lösungen, Suspensionen etc. Als Injectionsmedium kommt vorzugsweise Wasser zur Anwendung, welches die bei Injektionslösungen üblichern Zusätze wie Stabilisierungsmittel, Lösungsvermittler und Puffer enthält.

Derartige Zusätze sind z. B. Tartrat- und Citrat-Puffer, Äthanol, Komplexbildner (wie Äthylendiamintetraessigsäure und deren nichttoxische Salze), hochmolekulare Polymere (wie flüssiges Polyäthylenoxid) zur Viskositätsregelung. Flüssige Trägerstoffe für Injektionslösungen müssen steril sein und werden vorzugsweise in Ampullen abgefüllt. Feste Trägerstoffe sind z. B. Stärke, Lactose, Mannit, Methylcellulose, Talkum, hochdisperse Kieselsäuren, höhermolekulare Fettsäuren (wie Stearinsäure), Gelatin, Agar-Agar, Calciumphosphat, Magnesiumstearat, tierische und pflanzlische Fette, feste hochmolekulare Polymere (wie Polyäthylenglykole); für orale Applikation geeignete Zubereitungen können gewünschtenfalls Geschmacks- und Süßstoffe enthalten.

Die Dosierung kann von verschiedenen Faktoren, wie Applikationsweise, Spezies, Alter und / oder individuellem Zustand abhängen. Die täglich zu verabreichenden Dosen liegen bei etwa 1 - 1000 mg/Mensch, vorzugsweise bei 10 - 200 mg/Mensch und können auf einmal oder mehrere Male verteilt eingenommen werden.

Bevorzugt im Sinne der vorliegenden Erfindung sind außer den in den Beispielen genannten Verbindungen und durch Kombination aller in den Ansprüchen genannten Bedeutungen der Substituenten ableitbaren Verbindungen die folgenden Diphosphonsäure, sowie deren Natriumsalze, Methyl und Ethylester.

1-Hydroxy-2-(3-methyl-1.2.4-thiadiazol-5-yl)ethan-1.1-diphosphonsäure
1-Hydroxy-2-(3-phenyl-1.2.4-thiadiazol-5-yl)ethan-1.1-diphosphonsäure
1-Hydroxy-2-(3-cyclohexylmethyl-1.2.4-thiazidazol-5-yl)ethan-1.1-diphosphonsäure
1-Hydroxy-2-(3-methyl-isoxazol-5-yl)ethan-1.1-diphosphonsäure
1-Hydroxy-2-(3-phenyl-isoxazol-5-yl)ethan-1.1-diphosphonsäure
1-Hydroxy-2-(3-methyl-1.2.5-oxadiazol-4-yl)ethan-1.1-diphosphonsäure
1-Hydroxy-2-(2-methyl-1.3.4-oxadiazol-5-yl)ethan-1.1-diphosphonsäure
1-Hydroxy-2-(3-phenyl-1.2.4-oxadiaxol-5-yl)ethan-1.1-diphosphonsäure
1-Hydroxy-2-(1.2.3-thiadiazol-4-yl)ethan-1.1-diphosphonsäure
1-Hydroxy-2-(1.2.5-thiadiazol-4-yl)ethan-1.1-diphosphonsäure
1-Hydroxy-2-(4-oxazolin-2-yl)ethan-1.1-diphosphonsäure
1-Hydroxy-2-(5-methoxy-oxazol-4-yl)ethan-1.1-diphosphonsäure
1-Hydroxy-2-(5-ethoxy-oxazol-4-yl)ethan-1.1-diphosphonsäure
1-Hydroxy-2-(2-amino-oxazol-4-yl)ethan- 1-diphosphonsäure
1-Hydroxy-2-(2.5-dimethyl-oxazol-4-yl)ethan-1.1-diphosphonsäure
1-Hydroxy-2-(5-ethoxy-2-methyl-oxazol-4-yl)ethan-1.1-diphosphonsäure
1-Hydroxy-2-(2-methyl-1.3.4-oxadiazol-5-yl)ethan-1.1-diphosphonsäure
1-Hydroxy-2-(3-phenyl-1.2.4-oxadiazol-5-yl)ethan-1.1-diphosphonsäure
1-Hydroxy-2-(1.2.3-thiadiazol-5-yl)ethan-1.1-diphosphonsäure
1-Hydroxy-2-(4-methyl-1.2.3-thiadiazol-5-yl)ethan-1.1-diphosphonsäure
1-Hydroxy-2-(5-methyl-imidazol-4-yl)ethan-1.1-diphosphonsäure
2-(2-Methyl-thiazol-4-yl)ethan-1.1-diphosphonsäure
2-(2-Methyl-thiazol-4-yl)propan-1.1-diphosphonsäure
1-Hydroxy-2-(2-methyl-thiazol-5-yl)ethan-1.1-diphosphonsäure
2-(2-Methyl-thiazol-5-yl)propan-1.1-diphosphonsäure
1-Hydroxy-2-(1.2.3-triazol-4-yl)ethan-1.1-diphosphonsäure
1-Hydroxy-2-(1.2.4-triazol-3-yl)ethan-1.1-diphosphonsäure
1-Hydroxy-2-(2-amino-imidazol-4-yl)ethan-1.1-diphosphonsäure
2-(2-Methyl-thiazol-5-yl)ethan-1.1-diphosphonsäure
1-Hydroxy-2-(imidazol-4-yl)propan-1.1-diphosphonsäure

1-Amino-2-(imidazol-4-yl)ethan-1.1-diphosphonsäure
1-Dimethylamino-2-(imidazol-4-yl)ethan-1.1-diphosphonsäure
1-Hydroxy-2-(2-cyclohexylmethyl-1.3.4-oxadiazol-5-yl)ethan-1.1-diphosphonsäure
1-Hydroxy-2-(-2-cyclohexyl-1.3.4-oxadiazol-5-yl)ethan-1.1-diphosphonsäure
1-Hydroxy-2-(2-amino-thiazol-5-yl)ethan-1.1-diphosphonsäure
1-Hydroxy-2-(2-Chloro-thiazol-5-yl)ethan-1.1-diphosphonsäure
1-Hydroxy-2-(2-Chlor-oxazol-4-yl)ethan-1.1-diphosphonsäure
1-Hydroxy-2-(imidazol-2-yl)ethan-1.1-diphosphonsäure
1-Hydroxy-2-(1.2.4-triazol-1-yl)ethan-1.1-diphosphonsäure

Die nachfolgenden Beispiele zeigen einige der Verfahrensvarianten, die zur Synthese der erfindungsgemäßen Verbindungen verwendet werden können. Sie sollen jedoch nicht eine Einschränkung des Erfindungsgegenstandes darstellen. Die Verbindungen fallen in der Regel als hochschmelzende (Fp≧ 300° C) Festprodukte an (Mono- oder Dinatriumsalz), deren Struktur durch H-, P- und gegebenenfalls durch $^{13}C$ NMR-Spektroskopie gesichert wurde. Die Reinheit der Substanzen wurde mittels C,H,N,P,S, Na-Analyse

sowie durch Dünnschichtelektrophorese (Cellulose, Oxalat-Puffer von $P_H$ = 4.0) bestimmt. Zur Charakterisierung der einzelnen Verbindungen werden die $M_{rel}$-Werte (= relative Mobilität) bezogen auf Pyrophosphat ($M_{rel}$ = 1) angegeben.

Beispiel 1

1-Hydroxy-2-(imidazol-4-yl)ethan-1.1-diphosphonsäure

Zu 3,5g Imidazol-4-yl-essigsäure-hydrochlorid (Fp: 198 - 200 °C) in 40 ml Chlorbenzol gibt man 3 g phosphorige Säure, rührt 10. min. bei 110 °C, kühlt ab und tropft langsam 9 g Phosphortrichlorid zu. Man erhitzt 16 h auf 110 °C, kühlt ab, dekantiert das Chlorbenzol von einem orangefarbenen Sirup und versetzt den Rückstand mit 50 ml 6N Salzsäure. Die Suspension wird 5 h unter Rückfluß erhitzt, nach dem Abkühlen mit Kohle versetzt und abgesaugt.
Das Filtrat wird eingeengt, getrocknet und 2 h mit Aceton ausgekocht. Den Rückstand (4,3 g) löst man in 40 ml Wasser, bringt die Lösung mit 2N Natronlauge auf einen PH = 5, versetzt mit 50 ml Methanol und saugt den ausgefallenen Niederschlag ab.

Man erhält 1,2 g ≙ 16,9% d.Th. mit einem Fp > 290 °C. Die Verbindung fällt als Mononatriumsalz mit 2 Mol Kristallwasser an. ( $M_{rel}$ = 0,37 )

Beispiel 2

In analoger Weise wie in Beispiel 1 beschrieben erhält man durch Umsetzung von phoshoriger Säure und Phosphortrichlorid mit

a) 2-Methyl-thiazol-4-ylessigsäure (Fp: 119-121 °C; hergestellt durch Verseifung des entsprechenden Ethylesters ($Kp_{13}$: 127 °C), der nach J.Chem.Soc. 1946, 91 aus γ-Bromacetessigester durch Umsetzung mit Thioacetamid dargestellt wurde) die 1-Hydroxy-2-(2-methyl-thiazol-4-yl)ethan-1,1-diphosphonsäure, die als Dinatriumsalz mit 1 Mol Kristallwasser in einer Ausbeute von 57 % isoliert wurde; Fp >300 °C, $M_{rel}$ = 0.55.

b) (4-Imidazolin-2-yl)essigsäure (Fp: 108-110 °C; hergestellt durch Verseifung des Ethylesters (Fp: 102-105 °C), der durch Umsetzung von Ethylendiamin mit dem Iminoether des Cyanessigsäureethylesters dargestellt wurde) die 1-Hydroxy-2-(4-imidazolin-2-yl)ethan-1,1-diphosphonsäure,die als freie Säure mit 1 Mol Kristallwasser in einer Ausbeute von 14 % isoliert wurde; Fp: ca. 250 °C u.Z.; $M_{rel}$: 0.45.

c) 2-Amino-4-thiazolin-4-yl-essigsäure (Fp: 218-221 °C; hergestellt durch Verseifung des Ethylesters (ölige Substanz), der durch Umsetzung von Thiohanrstoff mit γ-Bromacetessigsäureethylester dargestellt wurde) die 2-(2-Amino4-thiazolin-4-yl)ethan-1-hydroxy-1,1-diphosphonsäure, die als freie Säure mit 2 Mol Kristallwasser in einer Ausbeute von 59 % isoliert wurde; Fp: 190-195 °C u.Z. ; $M_{rel}$: 0.40.

Beispiel 3

2-(1,2,5-Thiadiazol-4-yl)ethan-1,1-diphosphonsäuretetraethylester

Zu 0.2 g Natriumhydrid (69proz.) in 10 ml absolutem Toluol tropft man eine Lösung von 1.62 g Methandiphosphonsäuretetraethylester in 10 ml absolutem Toluol. Nach Beendigung der Wasserstoffentwicklung gibt man 1 g 4-Brommethyl-1,2,5-thiadiazol in 10 ml absolutem Toluol zu und läßt 12 Stunden bei Zimmermtemperatur rühren. Man gibt wenig Wasser zu, trennt die organische Phase ab, trocknet und engt ein. Der Rueckstand wird über einer Kieselgelsäule gereinigt (100 g; Fließmittel: Methylenchlorid/Methanol i.V. 98:2). Man erhält so 1.18 g = 55% eines farblosen Öls.

Beispiel 4

2-(1,2,5-Thiadiazol-4-yl)ethan-1,1-diphosphonsäure

1.18 g des in Beispiel 3 beschriebenen 2-(1,2,5-Thiadiazol-4-yl)ethan-1,1-diphosphonsäure-tetraethylesters werden unter Stickstoff mit 3.3 ml Trimethylbromsilan versetzt. Man läßt 24 Stunden bei Zimmertemperatur stehen, engt die Lösung ein, versetzt den Rückstand mit Wasser, stellt die Lösung mit NaOH auf einen pH = 5 und versetzt sie mit Methanol. Der ausgefallene Niederschlag wird abgesaugt. Man erhält so 0.56 g = 53 % der gewünschten Diphosphonsäure als Dinatriumsalz mit 1 Mol Krsitallwasser; Fp > 300 °C; $M_{rel}$: 0.9.

## Patentansprüche

**1.** Neue Alkyldiphosphonsäure-Derivate der allgemeinen Formel I

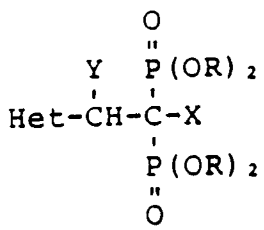

(I)

in der

Het einen Imidazol-, Imidazolin-, Isoxazol-, Oxazol-; Oxazolin-, Thiazol-, Thiazolin-, Triazol-, Oxadiazol- oder Thiadiazol- rest, der gegebenenfalls ein- oder mehrfach durch $C_1$-$C_4$-Alkyl, $C_1$-$C_4$-Alkoxy, Phenyl, Cyclohexyl, Cyclohexylmethyl, Halogen oder die Aminogruppe substituiert sein kann,

Y Wasserstoff oder $C_1$-$C_4$-Alkyl

X Wasserstoff, Hydroxy oder gegebenenfalls durch $C_1$-$C_4$-Aklyl substituiertes Amino und

R Wasserstoff oder $C_1$-$C_4$-Alkyl

bedeuten.

Sowie deren pharmakologisch unbedentliche Salze

**2.** Verfahren zur Herstellung von neuen Alkyldiphosphonsäure-Derivaten der allgemeinen Formel I

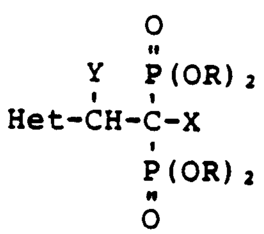

(I)

in der Het, Y, X, R die in Anspruch angegebenen Bedeutungen besitzen
sowie deren pharmakologisch unbedenkliche Salze,
dadurch gekennzeichnet, daß man

a) eine Carbonsäure der allgemeinen Formel II

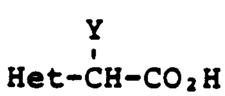

(II),

in der Het und Y die oben angegebenen Bedeutungen haben, mit einem Gemisch aus phosphoriger Säure oder Phosphorsäure und einem Phosphorhalogenid umgesetzt und anschließend zur freien Diphosphonsäure verseift, oder

b) ein Carbonsäurechlorid der allgemeinen Formel III

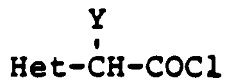

(III),

in der Het und Y die oben angegebenen Bedeutungen haben, mit einem Trialkylphosphit der allgemeinen Formel IV

$P(OR')_3$ (IV),

in der R′ niederes Alkyl bedeutet, zu einem Acylphosphonat der allgemeinen Formel V

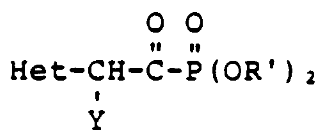

(V),

in der Het, Y und R′ die oben angegebenen Bedeutungen haben,
umsetzt, anschließend mit einem Dialkylphosphit der allgemeinen Formel VI

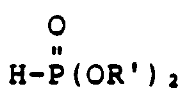

(VI),

in der R′ die oben angegebene Bedeutung hat, zu einem Diphosphonat der allgemeinen Formel VII

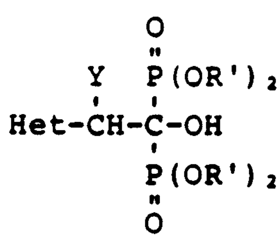

(VII),

in der Het, Y und R′ die oben angegebenen Bedeutungen haben, reagieren läßt und gegebenenfalls die entstandenen Tetraester zu Diestern oder Säuren der allgemeinen Formel I verseift, oder
für den Fall, daß X in der allgemeinen Formel I gegebenenfalls durch Alkylgruppen substituiertes Amino bedeutet,
c) ein Carbonsäurederivat der allgemeinen Formel VIII

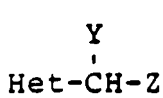

(VIII),

in der Het und Y die oben angegebenen Bedeutungen haben und Z eine Nitril-, Iminoether- oder eine N,N-Dialkylcarboxamidogruppe darstellt,

mit einer Phosphorverbindung der allgemeinen Formel IX

$PT_3$ (IX),

in der T = Halogen, OH oder OR′ bedeutet, wobei R′ die oben angegebene Bedeutung hat, umsetzt und gegebenenfalls anschließend verseift,
oder
für den Fall daß X in der allgemeinen Formel I Wasserstoff bedeutet,
d) eine Verbindung der allgemeinen Formel X

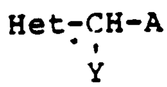
(X),

in der Het und Y die oben angegebenen Bedeutungen haben und A einen reaktiven Rest wie z. B. Halogen oder ein Sulfonat darstellt, mit einer Verbindung der allgemeinen Formel XI

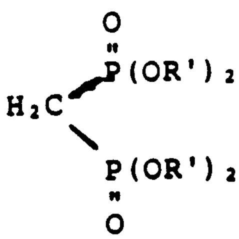
(XI),

in der R′ die oben angegebene Bedeutung hat, zu einem Diphosphonat der allgemeinen Formel XII

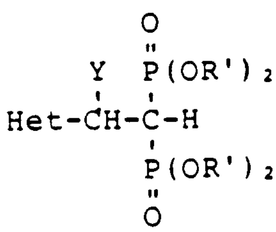
(XII),

in der Het, Y und R′ die oben angegebenen Bedeutungen haben,
umsetzt und gegebenenfalls die entstandenen Tetraester zu Diestern oder Säuren der allgemeinen Formel I verseift.

**3.** Arzneimittel, enthaltend eine Verbindung gemäß Anspruch 1, sowie übliche pharmakologisch unbedenkliche Träger-oder Hilfsstoffe.

**4.** Verwendung von Verbindungen gemäß Anspruch 1 zur Herstellung von Arzneimitteln für die Behandlung von Calciumstoffwechselstörungen.

**Claims**

**1.** New alkyldiphosphonic acid derivatives of the general formula I

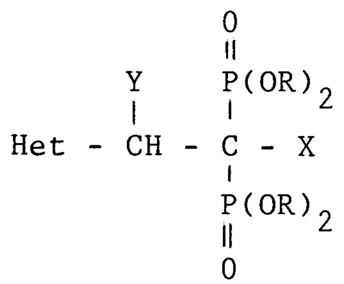

(I)

in which Het signifies an imidazole, imidazoline, isoxazole, oxazole, oxazoline, thiazole, thiazoline, triazole, oxadiazole or thiadiazole radical which can possibly be substituted one or more times by $C_1$-$C_4$-alkyl, $C_1$-$C_4$-alkoxy, phenyl, cyclohexyl, cyclohexylmethyl, halogen or the amino group, Y hydrogen or $C_1$-$C_4$-alkyl, X hydrogen, hydroxyl or amino possibly substituted by $C_1$-$C_4$-alkyl and R hydrogen or $C_1$-$C_4$-alkyl, as well as their pharmacologically acceptable salts.

**2.** Process for the preparation of new alkyldiphosphonic acid derivatives of the general formula I

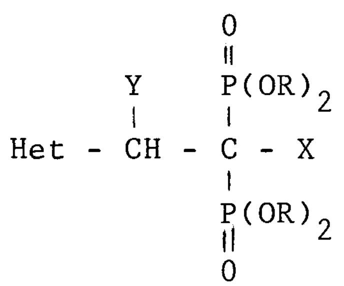

(I)

in which Het, Y, X, R possess the meanings given in claim 1, characterised in that one

a) reacts a carboxylic acid of the general formula II

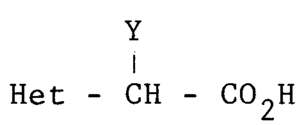

(II),

in which Het and Y have the above-given meanings, with a mixture of phosphorous acid or phosphoric acid and a phosphorus halide and subsequently saponifies to the free diphosphonic acid; or

b) reacts a carboxylic acid chloride of the general formula III

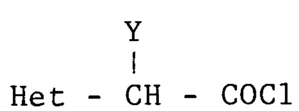

(III),

in which Het and Y have the above-given meanings, with a trialkyl phosphite of the general formula IV

$P(OR')_3$ (IV),

in which R' signifies lower alkyl, to an acyl phosphonate of the general formula V

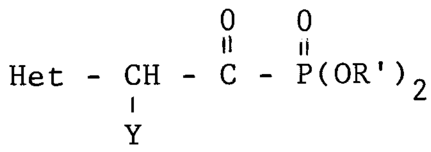

(V),

in which Het, Y and R' have the above-given meanings, subsequently allows to react with a dialkyl phosphite of the general formula VI

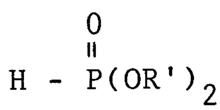

(VI),

in which R' has the above-given meaning, to a diphosphonate of the general formula VII

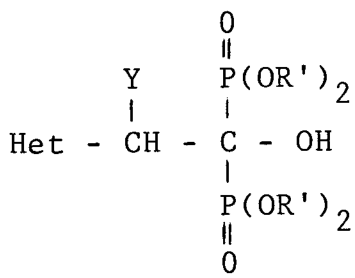

(VII)

in which Het, Y and R' have the above-given meanings, and possibly saponifies the resultant tetraesters to diesters or acids of the general formula I;
or, for the case that X in general formula I signifies amino possibly substituted by alkyl groups,
c) reacts a carboxylic acid derivative of the general formula VIII

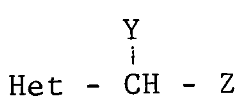

(VIII),

in which Het and Y have the above-given meanings and Z represents a nitrile, imino ether or an N,N-dialkylcarboxamido group, with a phosphorus compound of the general formula IX

$PT_3$ (IX).

in which T = halogen, OH or OR', whereby R' has the above-given meaning, and possibly subsequently saponifies;
or, for the case that X in general formula I signifies hydrogen,
d) reacts a compound of the general formula X

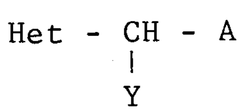

(X),

in which Het and Y have the above-given meanings and A represents a reactive residue, such as e.g. halogen or a sulphonate, with a compound of the general formula XI

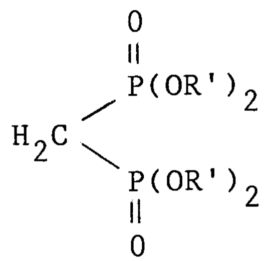

(XI).

in which R' has the above-given meaning, to a diphosphonate of the general formula XII

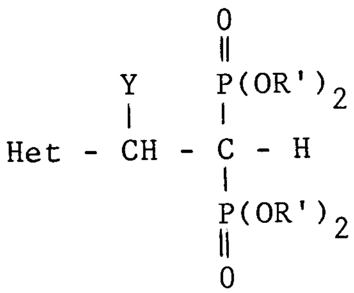

(XII),

in which Het, Y and R' have the above-given meanings, and possibly saponifies the resultant tetraesters to diesters or acids of general formula I.

**3.** Medicaments containing a compound according to claim 1, as well as usual pharmacologically acceptable carrier or adjuvant materials.

**4.** Use of compounds according to claim 1 for the preparation of medicaments for the treatment of calcium metabolism disturbances.

**Revendications**

**1.** Nouveaux dérivés d'acides alkyldiphosphoniques de formule générale I

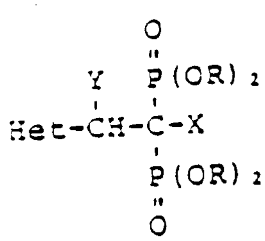

(I)

où
Het represente un groupe imidazole, imidazoline, isoxazole, oxazole, oxazoline, thiazole, thiazoline, triazole, oxadiazole, ou thiadiazole, qui peut être éventuellement substitué une ou plusieurs fois par un groupe alkyle en $C_1$-$C_4$, alcoxy en $C_1$-$C_4$, phényle, cyclohexyle, cyclohexylméthyle, halogene ou amino,
Y represente l'hydrogene ou un groupe alkyle en $C_1$-$C_4$,
X représente l'hydrogène un groupe hydroxy ou un groupe amino éventuellement substitué par un groupe alkyle en $C_1$-$C_4$, et
R represente l'hydrogene ou un groupe alkyle en $C_1$-$C_4$, ainsi que leurs sels pharmaceutiquement acceptables.

**2.** Procédé de préparation de nouveaux dérivés d'acides alkyldiphosphoniques de formule générale I

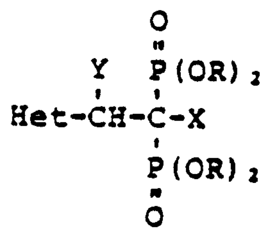

(I)

où Het, Y, X, R ont les significations mentionnées à la revendication 1. caracterise en ce que

a) on fait reagir un acide carcoxylique de formule generale II

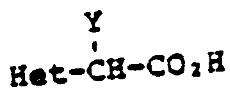

(II),

où Het et Y ont les significations mentionnées plus haut, avec un mélange d'acide phosphoreux ou d'acide phosphorique et d'un halogenure de phosphore, et ensuite, on saponifie en acide diphosphonique libre, ou

b) on fait réagir un chlorure d'acide carboxylique de formule générale III

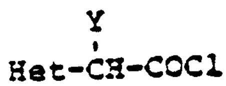

(III),

où Het et Y ont les significations mentionnées plus haut, avec un trialkylphosphite de formule generale IV

$P(OR')_3$ (IV),

ou R represente un groupe alkyle inferieur, en un phosphonate d'acyle de formule generale V

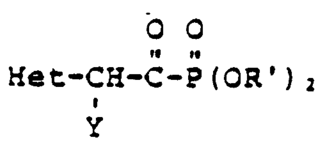

(V),

où Het, Y et R' ont les significations mentionnées plus haut,
ensuite on fait reagir le compose obtenu avec un dialkylphosphite de formule générale VI

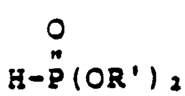

(VI),

où R' a la signification mentionnee plus haut, en un diphosphonate de formule générale VII

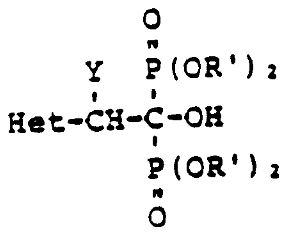 (VII),

ou Het, Y et R' ont les significations mentionnees plus haut, et eventuellement, on saponifie le tetraester produit en diester ou en acide de formule générale I,
ou
dans le cas ou X, dans la formule generale I, represente un groupe amino eventuellement substitué par un groupe alkyle,
c) on fait réagir un dérivé d'acide carboxylique de formule générale VIII

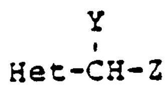 (VIII),

où Het et Y on les significations mentionnées plus haut, et Z représente un groupe nitrile, iminoéther ou N,N-dialkylcarboxyamido,
avec un compose du phosphore de formule generale IX

$PT_3$ (IX),

ou T = un halogene, un groupe OH ou OR' ou R' a la signification mentionnee plus haut, et ensuite on saponifie éventuellement,
ou
dans le cas où X, dans la formule générale I, représente l'hydrogène,
d) on fair reagir un compose de formule generale X

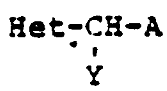 (X),

où Het et Y ont les significations mentionnees plus haut et A represente un groupe réactif comme par exemple un halogène ou un sulfonate, avec un composé de formule générale XI

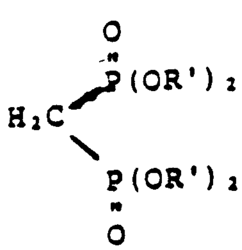 (XI),

ou R' a la signification mentionnee plus haut, en un diphosphonate de formule générale XII

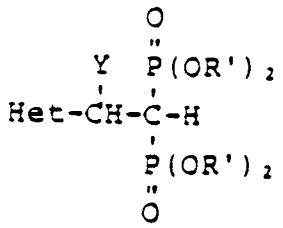

(XII),

ou Het, Y et R' ont les significations mentionnees plus haut, et on saponifie éventuellement le tétraester formé en diester ou en acide de formule générale I.

**3.** Medicament contenant un compose selon la revendication 1, ainsi que des supports ou adjuvants usuels pharmacologiquement acceptables.

**4.** Utilisation des composés selon la revendication 1, pour la préparation de médicaments pour le traitement de troubles du metabolisme du calcium.